# EUROPÄISCHE PATENTSCHRIFT

(11) **EP 2 163 936 B1**
(45) Veröffentlichungstag und Bekanntmachung des Hinweises auf die Patenterteilung: **12.12.2012**
(21) Anmeldenummer: 08016313.2
(22) Anmeldetag: 16.09.2008
(51) Int. Cl.: G02B 26/10, A61F 9/008, G02B 26/08

(54) **Vorrichtung zum Ablenken eines Laserstrahls**
Device for deflecting a laser beam
Dispositif destiné à la déviation d'un rayon laser

(43) Veröffentlichungstag der Anmeldung: 17.03.2010
(73) Patentinhaber: Ziemer Holding AG, 2562 Port (CH)
(72) Erfinder: Rathjen, Christian, 28197 Bremen (DE)
(74) Vertreter: Rentsch Partner AG

(56) Entgegenhaltungen:
- EP-A- 0 520 388
- EP-A- 1 557 710
- EP-A1- 1 279 386
- US-A1- 2005 046 936
- US-A1- 2005 228 366
- US-B1- 6 341 030

## Beschreibung

Technisches Gebiet Die vorliegende Erfindung betrifft eine Vorrichtung und ein Verfahren zum Ablenken eines Laserstrahls. Die vorliegende Erfindung betrifft insbesondere eine Vorrichtung und ein Verfahren zum Ablenken eines Laserstrahls mit einem um eine Drehachse auslenkbar angeordneten Ablenkspiegel.

### Stand der Technik

Zum Abtasten (Scannen) eines Bildbereiches respektive eines Arbeitsbereiches bei der Materialbearbeitung werden Lichtstrahlen (Laserstrahlen) mittels geeigneter Ablenkvorrichtungen (Scanner) in ein oder zwei Abtastrichtungen (x/y-Scanrichtung) abgelenkt. Vorzugsweise werden dabei zur Vermeidung von Vignettierung annähernd kollimierte Lichtstrahlen um das Zentrum der Eintrittspupille einer Fokussieroptik geschwenkt. Die Ablenkung der Lichtstrahlen wird im Allgemeinen mit schwenkbaren Spiegeln vorgenommen, beispielsweise mit Galvanoscannern, Piezoscannern. Polygonscannern oder Resonanzscannern.

WO200607489 beschreibt ein Laserbehandlungsgerät, welches einen Laserstrahl mit Hilfe von zwei auf Galvanoscannern montierten Spiegeln, und mit Zwischenabbildungen durch jeweils eine Relaisoptik, in die Eintrittspupille eines Objektivs abbildet.

EP1584310 beschreibt ein System mit zwei Scannachsen mit nur einer Relaisoptik, dafür aber mit einem zusätzlichen synchron bewegten Spiegel für die zweite Scannachse, der verhindert, dass der von der zweiten Scannachse abgelenkte Strahl die erste Drehachse ausserhalb ihres Drehzentrums triff.

EP 1 557 710 beschreibt einen optischen Scanner für Abbildungsgeräte wie Drucker, Köpierer oder Faxgerat, bei welchem der Lichtstrahl beim Abbildungsvorgang hinsichtlich einer zur Drehachse orthogonalen Ebene schräg auf einen Ablenkspiegel trifft. Gemäss EP 1 557 710 wird der Lichtstrahl mit einer reduzierten aber die Spiegelbreite übertreffenden Strahlbreite auf den Ablenkspiegel projiziert ("overfilled" Scanner). Um einer abhängig vom Ablenkungswinkel des Spiegels (durch axiale Rotation) verdrehten Abbildung des abgelenkten Lichtstrahls entgegenzuwirken, wird dadurch der abgelenkte Lichtstrahl in Abhängigkeit vom Drehwinkel beschnitten.

US 6,341,030 beschreibt einen weiteren "overfilled" Scanner für Abbildungsgeräte, bei welchem der Lichtstrahl ebenfalls schräg auf den Ablenkspiegel trifft, die als Facetten eines rotierenden Polygonspiegels ausgebildet sind, wobei die mit optischen strahlformenden Mitteln reduzierte Lichtstrahlbreite die Spiegelbreite der einzelnen Facetten in Scannrichtung übertrifft.

US 2005 228 366 beschreibt einen ophtalmologischen Scanner, der für Steuerung von fs-Laserpulsen geeignet ist.

Um hohe Scanngeschwindigkeiten zu erzeugen sind möglichst trägheitsarme Spiegel (d.h. insbesondere kleine Spiegel) erwünscht. Dabei lässt sich aufgrund fundamentaler optischer Gesetze bei gegebenen Abbildungsgrössen der Fokussieroptik (Fokusgrösse, Arbeitsabstand und Bildfeldhöhe) die Spiegelgrösse nicht verkleinern ohne damit gleichzeitig den Scannwinkel zu vergrössern. Umgekehrt muss für einen ebenfalls geschwindigkeitssteigernden kleineren Scannwinkel der Strahldurchmesser vergrössert werden. Formal ist das Produkt aus Strahldurchmesser und Scannwinkel eine Konstante des optischen Systems. Die im Sinne einer Geschwindigkeitserhöhung gleichzeitig gewünschte Verkleinerung des Spiegeldurchmessers und des Scannwinkels lässt sich prinzipbedingt nicht realisieren.

### Darstellung der Erfindung

Es ist eine Aufgabe der vorliegenden Erfindung eine Vorrichtung und ein Verfahren zum Ablenken eines Laserstrahls mit einem um eine Drehachse auslenkbaren Ablenkspiegel vorzuschlagen, welche zumindest einige Nachteile der bekannten Systeme nicht aufweisen. Es ist insbesondere eine Aufgabe der vorliegenden Erfindung eine Vorrichtung und ein Verfahren zum Ablenken eines Laserstrahls vorzuschlagen, welche hohe Scangeschwindigkeiten durch Grössenreduktion des Ablenkspiegels ermöglichen, ohne dafür eine Vergrösserung des Scannwinkels erforderlich zu machen.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass zum Ablenken eines Laserstrahls mit einem um eine Drehachse auslenkbar angeordneten Ablenkspiegel, dem Ablenkspiegel ein strahlformendes optisches Element vorgelagert wird, welches eingerichtet ist, den Laserstrahl mit einer in Richtung der Drehachse reduzierten Strahlbreite auf den Ablenkspiegel zu projizieren. Das heisst, das vorgelagerte strahlformende optische Element bewirkt eine Konvergierung des Laserstrahls auf eine zur Drehachse orthogonalen Längsachse des Ablenkspiegels. Bei einem Laserstrahl mit einem im Wesentlichen kreisrundem Strahlquerschnitt, bewirkt die Transformation des strahlformenden optischen Elements beispielsweise eine Projektion mit einem schmalen, länglichen elliptischen Querschnitt des Laserstrahls auf den Ablenkspiegel. Der Ablenkspiegel weist eine in Richtung der Drehachse orientierte Spiegelbreite auf, welche schmäler ist, als die unreduzierte Eingangsstrahlbreite des Laserstrahls vor dem strahlformenden optischen Element Die unreduzierte Eingangsstrahlbreite des Laserstrahls beträgt beispielsweise mindestens 15mm. Die reduzierte Strahlbreite beträgt auf dem Ablenkspiegel beispielsweise 5-25% der Eingangsstrahlbreite. In einer weniger bevorzugten Ausführungsvariante wird die Strahlbreite nicht bloss in Richtung der Drehachse reduziert, sondern der Laserstrahl wird konvergierend auf den Ablenkspiegel projiziert (Fokussierung). Dadurch lässt sich zwar ebenso ein kleinerer Ablenkspiegel verwenden, dafür muss jedoch die nachgeschaltete Optik mit einer breiteren Pupille versehen werden.

Durch die verschmälernde (konvergierende) Transformation des Laserstrahls in der orthogonal zur Abtastrichtung (Scanrichtung) liegenden Drehachse wird dem Umstand Rechnung getragen, dass die oben genannten prinzipbedingten Beschränkungen nur für die Abtastebene (Scanebene) gelten. Orthogonal zur Abtastebene, d.h. in der Richtung der Drehachse, kann der Lichtstrahl verformt werden, ohne diese fundamentalen Gesetzmässigkeit für Scannersysteme zu verletzen. Durch die Verschmälerung (Konvergierung) der Strahlbreite in der Richtung der Drehachse (orthogonal zur Abtastebene) kann somit ein Ablenkspiegel mit einer in der selben Richtung reduzierten Breite verwendet werden. Durch die reduzierten Abmessungen des Ablenkspiegels werden somit auch dessen Masse und Massenträgheit deutlich reduziert. Durch die Reduktion der Massenträgheit können kleinere und damit leichtere Scanantriebe mit geringerem Energiebedarf bei gleicher oder sogar höherer Scangeschwindigkeit verwendet werden.

Vorzugsweise ist dem Ablenkspiegel ein strahlformendes optisches Element nachgelagert, welches eingerichtet ist, den Laserstrahl vom Ablenkspiegel im Wesentlichen kollimiert, mit einer in Richtung der Drehachse der unreduzierten Eingangsstrahlbreite entsprechenden Strahlbreite weiter zu leiten. Da sich die in Richtung der Drehachse orientierte Breite des Laserstrahls vom vorgeschalteten ersten strahlformenden optischen Element zum Ablenkspiegel hin verjüngt (konvergierende Lichtstrahlen), weitet sich diese Laserstrahlbreite durch die Reflektion am Ablenkspiegel entsprechend wieder aus (divergierende reflektierte Lichtstrahlen). Das zweite, dem Ablenkspiegel nachgeschaltete strahlformende optische Element ist eingerichtet, die Strahlbreite in der Richtung der Drehachse zu schmälern und dadurch den Laserstrahl so zu formen, dass die sich ab dem Ablenkspiegel in Richtung der Drehachse ausweitende Laserstrahlbreite auf eine konstante Laserstrahlbreite transformiert wird. Das heisst, das nachgelagerte strahlformende optische Element bewirkt eine Konvergierung des vom Ablenkspiegel reflektierten und abgelenkten, aufgeweiteten Laserstrahls zur Kompensation der Strahlaufweitung. Das zweite strahlformende optische Element ist dabei so zum Ablenkspiegel positioniert (distanziert), dass der Querschnitt des austretenden Laserstrahls im Wesentlichen demjenigen des ursprünglichen untransformierten Laserstrahls vor dem ersten vorgeschalteten strahlformenden optischen Element entspricht. Das zweite, dem Ablenkspiegel nachgeschaltete strahlformende optische Element macht somit die Transformation des ersten, dem Ablenkspiegel vorgeschalteten strahlformenden optischen Elements wieder rückgängig, so dass der Laserstrahl vom zweiten strahlformenden optischen Element im Wesentlichen mit dem gleichen Strahlquerschnitt (Breite, Form) weitergeleitet wird, wie er ursprünglich beim ersten strahlformenden optischen Element eingetroffen ist. Bei einem Laserstrahl mit einem im Wesentlichen kreisrundem ursprünglichen Strahlquerschnitt, der mit einem elliptischen Querschnitt auf den Ablenkspiegel projiziert wird, formt das zweite strahlformende optische Element den Laserstrahl beispielsweise wiederum mit einem im Wesentlichen kreisrunden Querschnitt.

Je nach Ausführungsvariante ist das dem Ablenkspiegel vorgelagerte respektive das dem Ablenkspiegel nachgelagerte strahlformende optische Element als Zylinderlinse oder als diffraktives optisches Element ausgestaltet, wobei hier mit der Zylinderlinse stellvertretend auch refraktiv optische Elemente bezeichnet sein sollen, welche eine gleiche Wirkung wie eine Zylinderlinse oder eine einer Zylinderlinse entsprechende Wirkung mit verbesserter Korrektur von Aberrationen aufweisen.

Vorzugsweise weist der Ablenkspiegel eine im Wesentlichen elliptisch ausgestaltete spiegelnde Vorderseite auf, wobei die orthogonal zur Drehachse verlaufende Spiegellänge (Ellipsenlänge) der Vorderseite ein Mehrfaches der in Richtung der Drehachse verlaufenden Spiegelbreite (Ellipsenbreite) der Vorderseite beträgt

In einer Ausführungsvariante ist die von der Spiegelnden Vorderseite des Ablenkspiegels abgewandte Rückseite des Ablenkspiegels stromlinienförmig gerundet. Dadurch können durch Strömungswiderstand bedingte Leistungsverluste reduziert werden respektive bei gleich bleibender Leistung höhere Scangeschwindigkeiten (Scanfrequenzen) und/oder Auslenkungen (Amplituden) realisiert werden,

In einer weiteren Ausführungsvariante ist der Ablenkspiegel in der orthogonal zur Drehachse verlaufenden Längsrichtung versteift. Die Versteifung des Ablenkspiegels orthogonal zur Drehachse verhindert Biegeschwingungen des Ablenkspiegels, welche eine Deformation der Wellenfront des Laserstrahls bewirken würden.

Vorzugsweise ist der Ablenkspiegel mit einem Antrieb, insbesondere mit einem Oszillator, zur Auslenkung des Ablenkspiegels um die Drehachse gekoppelt. Vorzugsweise wird der Ablenkspiegel oszillierend um die Drehachse hin und her geschwenkt, Der Laserstrahl ist ein mit Femtosekundenlaserpulsen gepulster Laserstrahl, und dem Ablenkspiegel ist ein Lichtprojektor zur fokussierten Projektion der Femtosekundenlaserpulse für eine Auflösung von Augengewebe nachgeschaltet. Die Ablenkvorrichtung ist ein Teil einer ophthalmologischen Vorrichtung.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
- Figur 1:: zeigt schematisch im Querschnitt und in der Aufsicht eine Vorrichtung zum Ablenken eines Laserstrahls mit einem Ablenkspiegel gemäss dem Stand der Technik.
- Figur 2:: zeigt schematisch im Querschnitt und in der Aufsicht eine erfindungsgemässe Ausführung einer Vorrichtung zum Ablenken eines Laserstrahls mit einem Ablenkspiegel.
- Figur 3:: zeigt schematisch eine kombinierte Ansicht des Strahlengangs aus zwei Perspektiven, welche die Veränderung der Strahlbreite in Bezug zur Drehachse des Ablenkspiegels erkennbar machen.
- Figur 4:: zeigt beispielhaft Querschnitte des Ablenkspiegels mit stromlinienförmig ausgestalteter Rückseite.

### Wege zur Ausführung der Erfindung

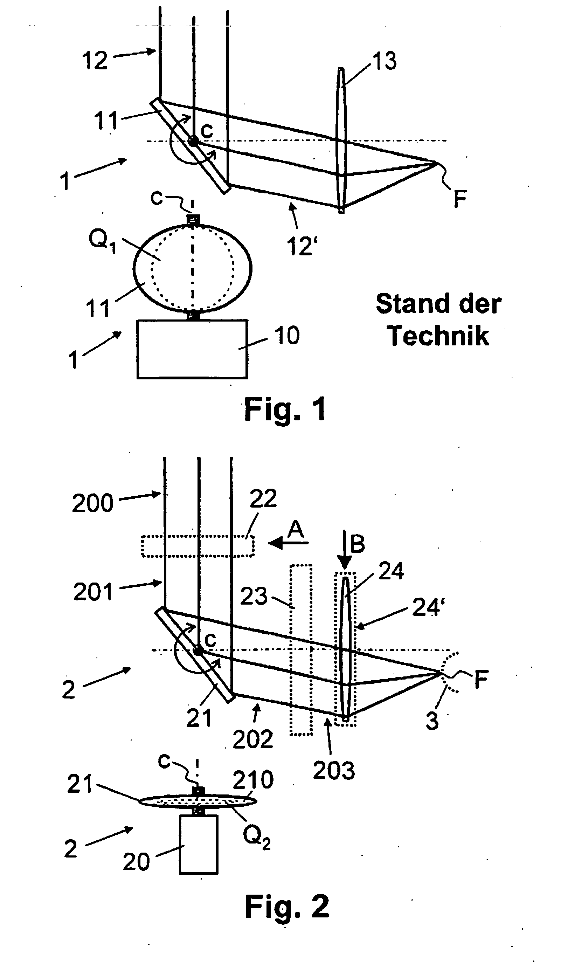

In der Figur 1 bezieht sich das Bezugszeichen 1 jeweils auf eine Ablenkvorrichtung gemäss dem Stand der Technik, welche im oberen Teil der Figur schematisch im Querschnitt und im unteren Teil der Figur schematisch in der Aufsicht dargestellt ist. Die Ablenkvorrichtung 1 umfasst einen um die Drehachse c auslenkbaren Ablenkspiegel 11. Der Ablenkspiegel 11 ist mit einem Antrieb 10 gekoppelt, welche den Ablenkspiegel 11 auslenkt, so dass der eingehende, im Wesentlichen kollimierte Laserstrahl 12, in einer orthogonal zur Drehachse c angeordneten Abtast- oder Scanebene abgelenkt wird. Der abgelenkte Lichtstrahl 12' wird durch einen dem Ablenkspiegel 11 nachgelagerten Lichtprojektor 13 auf einen Fokuspunkt F projiziert. Wie im unteren Teil der Figur 1 ersichtlich ist, weist der lichtstrahl einen im Wesentlichen kreisrunden Querschnitt Q₁ auf. Der Ablenkspiegel 11 ist elliptisch ausgestaltet und weist in der Richtung der Drehachse c eine Spiegelbreite auf, die mindestens der Strahlbreite des Lichtstrahls 12 entspricht.

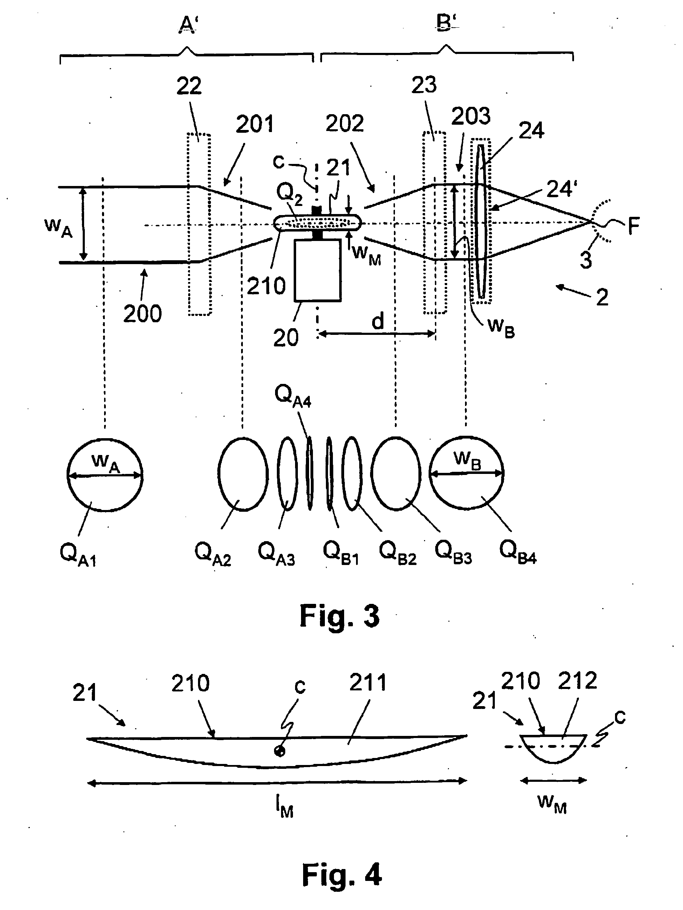

In den Figuren 2 und 3 bezieht sich das Bezugszeichen 2 jeweils auf eine erfindungsgemässe Ausführung, einer Ablenkvorrichtung zum Ablenken eines im Wesentlichen kollimierten Lichtstrahls, insbesondere eines Laserstrahls 200. Der Laserstrahl 200 ist ein mit Femtosekundenlaserpulsen gepulster Laserstrahl.

Die Ablenkvorrichtung 2 umfasst einen um die Drehachse c auslenkbaren Ablenkspiegel 21, der mit einem Antrieb 20 gekoppelt ist Der Antrieb 20 ist eingerichtet, den Ablenkspiegel 21 um die Drehachse c auszulenken, vorzugsweise in einer oszillierenden (hin und her schwenkenden) Bewegung. Der Antrieb 20 ist beispielsweise als Oszillator ausgeführt, zB. mit Hilfe eines Piezo- oder Galvanoantriebs.

Die Ablenkvorrichtung 2 umfasst zudem ein strahlformendes optisches Element 22, das dem Ablenkspiegel 21 vorgelagert angeordnet ist Das vorgeschaltete strahlformende optische Element 22 ist beispielsweise als Zylinderlinse (einschliesslich refraktiv optische Elemente mit gleicher Wirkung wie eine Zylinderlinse und eventuell verbesserter Korrektur von Aberrationen) oder als diffraktives optisches Element ausgestaltet und so ausgeführt, dass die ursprüngliche Strahlbreite w_{A} (Eingangsstrahlbreite) des eingehenden Laserstrahls 200 in der Richtung der Drehachse c reduziert wird, das heisst auf eine zur Drehachse c orthogonal verlaufenden Längsachse des Ablenkspiegels 21 konvergiert wird.

Im Bereich A' der Figur 3 ist die Ansicht des Laserstrahls aus der Perspektive dargestellt, die in der Figur 2 mit dem orthogonal zur Drehachse c verlaufenden Pfeil A angegeben ist Wie in der Figur 3 ersichtlich ist, weist der durch das strahlformende optische Element 22 geformte Laserstrahl 201 in Richtung der Drehachse c eine sich verjüngende (konvergierende) Strahlbreite auf. Das heisst, das strahlformende optische Element 22 verschmälert die Strahlbreite in Richtung der Drehachse c so, dass der geformte (konvergierende) Laserstrahl 201 mit einem elliptischen Querschnitt Q₂ auf den Ablenkspiegel 21 projiziert wird. Ausgehend von einem ursprünglichen, im Wesentlichen kreisrunden Querschnitt O_{A1}, des eingehenden Laserstrahls 200, verjüngt sich die in Richtung der Drehachse c orientierte Strahlbreite, so dass der geformte Laserstrahl 201 einen elliptischen Querschnitt Q_{A2}, Q_{A3}, Q_{A4} aufweist mit einer im Wesentlichen konstanten, der ursprünglichen Strahlbreite w_{A} des eingehenden Laserstrahls 200 entsprechenden Ellipsenlänge, und mit einer sich zum Ablenkspiegel 21 hin stetig reduzierenden Ellipsenrespektive Strahlbreite. Die in Richtung der Drehachse c orientierte Strahlbreite des auf den Ablenkspiegel 21 projizierten geformten Laserstrahls 201 beträgt beispielsweise zwischen 5-20% der ursprünglichen Strahlbreite w_{A}.

Der Ablenkspiegel 21 weist eine längliche, beispielsweise elliptisch ausgestaltete spiegelnde Vorderseite 210 auf. Die orthogonal zur Drehachse c verlaufende Spiegellänge IM (Ellipsenlänge) der Vorderseite 210 beträgt ein Mehrfaches der in Richtung der Drehachse c orientierten Spiegelbreite w_{M} (Ellipsenbreite) der Vorderseite 210. Die in Richtung der Drehachse c orientierte Spiegelbreite w_{M} des Ablenkspiegels 21 ist entsprechend der reduzierten Strahlbreite des auf den Ablenkspiegel 21 projizierten geformten Laserstrahls 201 schmäler als die ursprüngliche Strahlbreite w_{A} des eingehenden Laserstrahls 200. Zum Beispiel beträgt die in Richtung der Drehachse c orientierte Spiegelbreite w_{M} des Ablenkspiegels 21 5-25% der ursprünglichen Strahlbreite w_{A}. Wie in der Figur 4 durch den entlang der Längsachse verlaufenden Querschnitt 211 und den durch die Drehachse c verlaufenden Querschnitt 212 schematisch dargestellt ist, ist von der Vorderseite 210 abgewandte Rückseite des Ablenkspiegels 21 stromlinienförmig gerundet. Durch die Rundung der Rückseite wird der Ablenkspiegel 21 zudem in seiner Längsrichtung versteift.

Im Bereich B' der Figur 3 ist die Ansicht des Laserstrahls aus der Perspektive dargestellt, die in der Figur 2 mit dem orthogonal zur Drehachse c verlaufenden Pfeil B angegeben ist Wie in der Figur 3 ersichtlich ist, verbreitert sich die in Richtung der Drehachse c orientierte Strahlbreite auf Grund der Reflektion am Ablenkspiegel 21 stetig mit zunehmender Distanz vom Ablenkspiegel 21 (divergierende Lichtstrahlen). Das heisst der vom Ablenkspiegel 21 reflektierte und abgelenkte Laserstrahl 202 weist einen elliptischen Querschnitt Q_{B1}, Q_{B2}, Q_{B3} auf, der eine im Wesentlichen konstante, der ursprünglichen Strahlbreite w_{A} des eingehenden Laserstrahls 200 entsprechende Ellipsenlänge, und eine ausgehend vom auf dem Ablenkspiegel 21 projizierten elliptischen Querschnitt Q₂ stetig zunehmende Ellipsenrespektive Strahlbreite in Richtung der Drehachse c hat.

Die Ablenkvorrichtung 2 umfasst zudem ein weiteres strahlformendes optisches Element 23, das dem Ablenkspiegel 21 nachgelagert angeordnet ist. Das nachgeschaltete strahlformende optische Element 23 ist beispielsweise als Zylinderlinse (einschliesslich refraktiv optische Elemente mit gleicher Wirkung wie eine Zylinderlinse und eventuell verbesserter Korrektur von Aberrationen) oder als diffraktives optisches Element ausgestaltet und so ausgeführt, dass die zunehmende in Richtung der Drehachse c orientierte Strahlbreite des abgelenkten Laserstrahls 202 wieder auf eine konstante in Richtung der Drehachse c orientierte Strahlbreite reduziert wird. Wie in der Figur 3 schematisch illustriert ist, ist das strahlformende optische Element 23 mit einer Distanz d zum Ablenkspiegel 21 angeordnet, bei der die in Richtung der Drehachse c orientierte Strahlbreite des abgelenkten Laserstrahls 202 wieder der ursprünglichen Strahlbreite w_{A} des eingehenden Laserstrahls 200 entspricht. Das strahlformende optische Element 23 reduziert somit die in Richtung der Drehachse c orientierte Strahlbreite des abgelenkten Laserstrahls 202 auf eine konstante Strahlbreite wₛ, die der ursprünglichen Strahlbreite wₐ des eingehenden Laserstrahls 200 entspricht. Das strahlformende optische Element 23 führt den im Wesentlichen kollimierten Laserstrahl 203 dem nachgelagerten Lichtprojektor 24 zu, mit einem im Wesentlichen kreisrunden Querschnitt Q_{B4}, der dem ursprünglichen Querschnitt Q_{A1} des eingehenden Laserstrahls 200 entspricht.

Der Lichtprojektor 24 ist zB. eine Projektions- oder Fokussieroptik mit einer oder mehreren Projektionslinsen, welche den kollimierten Laserstrahl 203 fokussiert auf einen Fokuspunkt F projiziert. Die Ablenkvorrichtung 2 ist in eine ophthalmologische Vorrichtung integriert und der Lichtprojektor 24 ist eingerichtet die kollimierten Femtosekundenlaserpulse, für eine punktuelle Auflösung von Augengewebe 3, wie in der Figur 3 schematisch dargestellt, auf einen Fokuspunkt F in oder auf dem Augengewebe 3 zu projizieren.

An dieser Stelle soll zudem festgehalten werden, dass in weiteren Ausführungsvarianten weitere optische Komponenten in den Strahlengang zwischen den strahlformenden optischen Elementen 22 und 23 angeordnet sind, beispielsweise strahlablenkende optische Elemente für eine zusätzliche orthogonale Ablenkung des Laserstrahls (konventionelle xy-Doppelscanner-Anordnung). Weiterhin ist eine Ablenkung in Polarkoordinaten denkbar, wenn zusätzlich ein Bildrotator verwendet wird. Eine Ablenkung in zwei zueinander orthogonalen Ablenkrichtungen kann auch durch eine Kaskadierung von zwei Ablenkvorrichtungen 2 erreicht werden, die jeweils für eine Ablenkung des Laserstrahls in eine Ablenkrichtung vorgesehen sind. Zur Abbildung des abgelenkten Laserstrahls in die Eintrittspupille der Projektions- oder Fokussieroptik eines Lichtprojektors oder einer nachgelagerten Ablenkvorrichtung können zudem auch Relaisoptiken für Zwischenabbildungen vorgesehen werden, wie dies beispielsweise in W02006074898 und EP1584310 gezeigt wird.

Für eine Verstellung des Fokus in der Projektionsrichtung z wird entweder der Lichtprojektor 24 verstellt oder die Divergenz des Laserstrahls am Eingang des Scannersystems.

Mögliche Wellenfrontfehler der strahlformenden optischen Elemente 22, 23 werden in einer Ausführungsvariante durch vor- oder nachgeschaltete Kompensationselemente ausgeglichen, wobei die Kompensationselemente in einer weiteren Variante auch Teil der strahlformenden Elemente 23, 24 sind.

In einer Ausführungsvariante ist das strahlformende optische Element 23 in einem Abstand angeordnet und mit einer Brechkraft ausgestattet, dass entweder ein von der ursprünglichen Strahlbreite w_{A} des eingehenden Laserstrahls 200 verschiedener Strahldurchmesser oder eine fokussierende Wirkung erreicht wird. Beispielsweise kann, wie in den Figuren 2 und 3 schematisch illustriert, das sphärische Element des Lichtprojektors 24 durch ein weiteres strahlformendes optisches Element 24', z.B. in Form einer Zylinderlinse (einschliesslich refraktiv optische Elemente mit gleicher Wirkung wie eine Zylinderlinse und eventuell verbesserter Korrektur von Aberrationen), ersetzt werden, dessen Achse orthogonal zum strahlformenden optischen Element 23 ausgerichtet ist. Wird die Brechkraft des Elements 23 entsprechend erhöht, dann ergibt sich in Kombination mit dem weiteren strahlformenden Element 24' die gleiche Fokussierwirkung wie mit dem sphärischen Element des Lichtprojektors 24. Somit wird das Element 23 funktional Teil des Lichtprojektors 24.

Die Bildfelder des Lichtprojektors 24 können eben oder zur Applikation auf eine Hornhaut (Cornea) auch gekrümmt sein.

## Patentansprüche

1. Vorrichtung (2) zum Ablenken eines Laserstrahls (200) mit einem um eine Drehachse (c) auslenkbar angeordneten Ablenkspiegel (21) in einer orthogonal zur Drehachse (c) angeordneten Abtastebene und einem dem Ablenkspiegel (21) nachgeschalteten Lichtprojektor (24) zur fokussierten Projektion von Femtosekundenlaserpulsen des Laserstrahls (200) für eine Auflösung von Augengewebe (3), **dadurch gekennzeichnet,**
**dass** dem Ablenkspiegel (21) ein strahlformendes optisches Element (22) vorgelagert ist, welches eingerichtet ist, den Laserstrahl (200) auf eine zur Drehachse (c) orthogonale Längsachse des Ablenkspiegels (21) hin zu konvergieren und mit einer in Richtung der Drehachse (c) reduzierten Strahlbreite auf den Ablenkspiegel (21) zu projizieren, so dass der Laserstrahl (200) auf dem Ablenkspiegel (21) eine reduzierte Strahlbreite aufweist, und
**dass** der Ablenkspiegel (21) eine in Richtung der Drehachse (c) orientierte Spiegelbreite (w_{M}) aufweist, welche schmäler ist, als eine unreduzierte Eingangsstrahlbreite (w_{A}) des Laserstrahls (200) vor dem strahlformenden optischen Element (22).

2. Vorrichtung (2) nach Anspruch 1, **dadurch gekennzeichnet, dass** dem Ablenkspiegel (21) ein strahlformendes optisches Element (23) nachgelagert ist, welches eingerichtet ist, den Laserstrahl (202) vom Ablenkspiegel (21) im Wesentlichen kollimiert, mit einer in Richtung der Drehachse (c) der unreduzierten Eingangsstrahlbreite (w_{A}) entsprechenden Strahlbreite weiter zu leiten.

3. Vorrichtung (2) nach Anspruch 1, **dadurch gekennzeichnet, dass** dem Ablenkspiegel (21) mehrere strahlformende optische Elemente (23, 24') nachgelagert sind, welche so eingerichtet und angeordnet sind, dass der Laserstrahl (202) vom Ablenkspiegel (21) im Wesentlichen kollimiert und mit einer von der unreduzierten Eingangsstrahlbreite (w_{A}) abweichenden Strahlbreite, oder auf einen Fokuspunkt (F) konvergierend weitergeleitet wird.

4. Vorrichtung (2) nach einem der Ansprüche 2 oder 3, **dadurch gekennzeichnet, dass** das dem Ablenkspiegel (21) nachgelagerte strahlformende optische Element (23, 24') als Zylinderlinse oder diffraktives optisches Element ausgestaltet ist.

5. Vorrichtung (2) nach einem der Ansprüche 1 bis 4, **dadurch gekennzeichnet, dass** der Ablenkspiegel (21) eine im Wesentlichen elliptisch ausgestaltete spiegelnde Vorderseite (210) aufweist, wobei eine orthogonal zur Drehachse (c) verlaufende Spiegellänge (I_{M}) der Vorderseite (210) ein Mehrfaches einer in Richtung der Drehachse (c) orientierten Spiegelbreite (w_{M}) der Vorderseite (210) beträgt.

6. Vorrichtung (2) nach einem der Ansprüche 1 bis 5, **dadurch gekennzeichnet, dass** eine von einer spiegelnden Vorderseite (210) des Ablenkspiegels (21) abgewandte Rückseite des Ablenkspiegels (21) stromlinienförmig gerundet ist.

7. Vorrichtung (2) nach einem der Ansprüche 1 bis 6, **dadurch gekennzeichnet, dass** der Ablenkspiegel (21) in einer orthogonal zur Drehachse (c) verlaufenden Längsrichtung versteift ist.

8. Vorrichtung (2) nach einem der Ansprüche 1 bis 7, **dadurch gekennzeichnet, dass** das dem Ablenkspiegel (21) vorgelagerte strahlformende optische Element (22) als Zylinderlinse oder diffraktives optisches Element ausgestaltet ist.

9. Vorrichtung (2) nach einem der Ansprüche 1 bis 8, **dadurch gekennzeichnet, dass** die unreduzierte Eingangsstrahlbreite (w_{A}) des Laserstrahls mindestens 15mm beträgt.

10. Vorrichtung (2) nach einem der Ansprüche 1 bis 9, **gekennzeichnet durch** einen mit dem Ablenkspiegel (21) gekoppelten Antrieb (20), zur Auslenkung des Ablenkspiegels (21) um die Drehachse (c).

11. Vorrichtung (2) nach einem der Ansprüche 1 bis 10, **gekennzeichnet durch** einen mit dem Ablenkspiegel (21) gekoppelten Oszillator, zur oszillierenden Auslenkung des Ablenkspiegels (21) um die Drehachse (c).

12. Vorrichtung (2) nach einem der Ansprüche 1 bis 11, **gekennzeichnet durch** eine dem Ablenkspiegel (21) nachgelagerte Ablenkvorrichtung für eine Ablenkung des Laserstrahls in eine zur Ablenkrichtung des Ablenkspiegels (21) orthogonale zweite Ablenkrichtung, und eine Relaisoptik zur Abbildung des vom Ablenkspiegel (21) abgelenkten Laserstrahls in eine Eintrittspupille der nachgelagerten Ablenkvorrichtung.

## Claims

1. Device (2) for deflecting a laser beam (200) with a deflection mirror (21) arranged displaceably about a rotation axis (c) in a scanning plane arranged orthogonally with respect to the rotation axis (c) and a light projector (24) for the focused projection of femtosecond laser pulses of the laser beam (200) for a resolution of eye tissue (3), said projector being disposed downstream of the deflection mirror (21), **characterized**
**in that** a beam-shaping optical element (22) is disposed upstream of the deflection mirror (21), said optical element being designed to converge the laser beam (200) towards a longitudinal axis of the deflection mirror (21) orthogonal to the rotation axis (c) and to project said laser beam onto the deflection mirror (21) with a beam width that is reduced in the direction of the rotation axis (c), such that the laser beam (200) has a reduced beam width on the deflection mirror (21), and
**in that** the deflection mirror (21) has a mirror width (w_{M}), which is oriented in the direction of the rotation axis (c) and which is narrower than an unreduced input beam width (w_{A}) of the laser beam (200) upstream of the beam-shaping optical element (22).

2. Device (2) according to Claim 1, **characterized in that** a beam-shaping optical element (23) is disposed downstream of the deflection mirror (21), said optical element being designed to forward the laser beam (202) from the deflection mirror (21) substantially collimated, with a beam width corresponding to the unreduced input beam width (w_{A}) in the direction of the rotation axis (c).

3. Device (2) according to Claim 1, **characterized in that** a plurality of beam-shaping optical elements (23, 24') are disposed downstream of the deflection mirror (21), said optical elements being designed and arranged such that the laser beam (202) from the deflection mirror (21) is forwarded in a manner substantially collimated and with a beam width deviating from the unreduced input beam width (w_{A}), or convergently to a focal point (F).

4. Device (2) according to either of Claims 2 and 3, **characterized in that** the beam-shaping optical element (23, 24') disposed downstream of the deflection mirror (21) is configured as a cylindrical lens or diffractive optical element.

5. Device (2) according to one of Claims 1 to 4, **characterized in that** the deflection mirror (21) has a reflective front side (210) configured substantially elliptically, a mirror length (I_{M}) of the front side (210) that runs orthogonally with respect to the rotation axis (c) being a multiple of a mirror width (w_{M}) of the front side (210) that is oriented in the direction of the rotation axis (c).

6. Device (2) according to one of Claims 1 to 5, **characterized in that** a rear side of the deflection mirror (21) that is remote from a reflective front side (21 0) of the deflection mirror (21) is rounded in streamlined fashion.

7. Device (2) according to one of Claims 1 to 6, **characterized in that** the deflection mirror (21) is stiffened in a longitudinal direction running orthogonally with respect to the rotation axis (c).

8. Device (2) according to one of Claims 1 to 7, **characterized in that** the beam-shaping optical element (22) disposed upstream of the deflection mirror (21) is configured as a cylindrical lens or diffractive optical element.

9. Device (2) according to one of Claims 1 to 8, **characterized in that** the unreduced input beam width (w_{A}) of the laser beam is at least 15 mm.

10. Device (2) according to one of Claims 1 to 9, **characterized by** a drive (20) coupled to the deflection mirror (21) for displacing the deflection mirror (21) about the rotation axis (c).

11. Device (2) according to one of Claims 1 to 10, **characterized by** an oscillator coupled to the deflection mirror (21), for oscillating displacement of the deflection mirror (21) about the rotation axis (c).

12. Device (2) according to one of Claims 1 to 11, **characterized by** a deflection device disposed downstream of the deflection mirror (21) and serving for a deflection of the laser beam in a second deflection direction orthogonal to the deflection direction of the deflection mirror (21), and a relay optical unit for imaging the laser beam deflected by the deflection mirror (21) into an entrance pupil of the deflection device disposed downstream.

## Revendications

1. Dispositif (2) destiné à la déviation d'un rayon laser (200) avec un miroir de déviation (21) disposé de manière à pouvoir pivoter autour d'un axe de rotation (c) dans un plan de balayage disposé orthogonalement à l'axe de rotation (c) et avec un projecteur de lumière (24) branché derrière le miroir de déviation (21) pour la projection focalisée d'impulsions laser de femto secondes du faisceau laser (200) pour une résolution du tissus oculaire (3), **caractérisé**
**en ce que** devant le miroir de déviation (21) est placé un élément optique (22) formant le faisceau, qui est installé pour faire converger le faisceau laser (200) vers un axe longitudinal du miroir de déviation (21) orthogonal à l'axe de rotation (c) et pour le projeter sur le miroir de déviation (21) avec une largeur de faisceau réduite dans la direction de l'axe de rotation (c), de telle sorte que le faisceau laser (200) présente une largeur de faisceau réduite sur le miroir de déviation (21), et
**en ce que** le miroir de déviation (21) présente une largeur de miroir (w_{M}) orientée dans la direction de l'axe de rotation (c) qui est plus étroite qu'une largeur du faisceau d'entrée (w_{A}) non réduite du faisceau laser (200) avant l'élément optique (22) formant le faisceau.

2. Dispositif (2) selon la revendication 1, **caractérisé en ce que** derrière le miroir de déviation (21) est placé un élément optique (23) formant le faisceau, qui est installé pour collimater l'essentiel du faisceau laser (202) provenant du miroir de déviation (21) pour transmettre avec une largeur de faisceau correspondant à la largeur du faisceau d'entrée (w_{A}) non réduite dans la direction de l'axe de rotation (c).

3. Dispositif (2) selon la revendication 1, **caractérisé en ce que** derrière le miroir de déviation (21) sont placés plusieurs éléments optiques (23, 24') formant le faisceau, qui sont installés et disposés de manière à collimater essentiellement le faisceau laser (202) provenant du miroir de déviation (21) et avec une largeur de faisceau différente de la largeur de faisceau d'entrée (wA) non réduite ou pour le transmettre de manière à converger sur un point focal (F).

4. Dispositif (2) selon une des revendications 2 ou 3, **caractérisé en ce que** l'élément optique (23, 24') formant le faisceau, placé derrière le miroir de déviation (21), est sous la forme d'une lentille cylindrique ou d'un élément optique diffractif.

5. Dispositif (2) selon une des revendications 1 à 4, **caractérisé en ce que** le miroir de déviation (21) comporte une face avant (210) formée essentiellement d'une surface elliptique réfléchissante, dans lequel une longueur de miroir (I_{M}) de la face avant (210) orthogonale à l'axe de rotation (c) représente un multiple d'une largeur de miroir (w_{M}) de la face avant (210) orientée dans la direction de l'axe de rotation (c).

6. Dispositif (2) selon une des revendications 1 à 5, **caractérisé en ce qu'**une face arrière du miroir de déviation (21) éloignée de la face avant (210) réfléchissante du miroir de déviation (21) est arrondie en forme de lignes de flux.

7. Dispositif (2) selon une des revendications 1 à 6, **caractérisé en ce que** le miroir de déviation (21) est rigidifié dans une direction longitudinale orthogonale à l'axe de rotation (c).

8. Dispositif (2) selon une des revendications 1 à 7, **caractérisé en ce que** l'avant du miroir de déviation (21) est équipé d'éléments optiques (22) formant le faisceau sous forme de lentille cylindrique ou d'un élément optique diffractif.

9. Dispositif (2) selon une des revendications 1 à 8, **caractérisé en ce que** la largeur du faisceau d'entrée (w_{A}) non réduite du faisceau laser mesure au moins 15 mm.

10. Dispositif (2) selon une des revendications 1 à 9, **caractérisé par** une commande (20) accouplée au miroir de déviation (21) pour le pivotement du miroir de déviation (21) autour de l'axe de rotation (c).

11. Dispositif (2) selon une des revendications 1 à 10, **caractérisé par** un oscillateur accouplée au miroir de déviation (21) pour le pivotement oscillatoire du miroir de déviation (21) autour de l'axe de rotation (c).

12. Dispositif (2) selon une des revendications 1 à 11, **caractérisé par** un dispositif de déviation placé derrière le miroir de déviation (21) pour une déviation du faisceau laser dans une deuxième direction de déviation orthogonale à la direction de déviation du miroir de déviation (21), et une optique relais pour la reproduction du faisceau laser déviée par le miroir de déviation (21) dans une pupille d'entrée du dispositif de déviation placé derrière.
